# EUROPÄISCHE PATENTSCHRIFT

(11) **EP 2 678 466 B1**
(45) Veröffentlichungstag und Bekanntmachung des Hinweises auf die Patenterteilung: **03.02.2016**
(21) Anmeldenummer: 12710865.2
(22) Anmeldetag: 27.02.2012
(51) Int. Cl.: A61F 2/07, A61F 2/90, A61F 2/86, A61L 31/10, A61L 31/14, A61L 31/16, D01D 5/00, D01F 6/70, A61F 2/82, A61F 2/95

(54) **IMPLANTAT MIT FASERVLIES**
IMPLANT COMPRISING A NON-WOVEN FABRIC
IMPLANT ÉQUIPÉ DE NON-TISSÉ

(30) Priorität: 25.02.2011 DE 102011012501
(43) Veröffentlichungstag der Anmeldung: 01.01.2014
(73) Patentinhaber: Phenox GmbH, 44801 Bochum (DE)
(72) Erfinder: HANNES, Ralf, 44137 Dortmund (DE); MONSTADT, Hermann, 44797 Bochum (DE)
(74) Vertreter: Thiel, Christian
(86) Internationale Anmeldenummer: PCT/EP2012/000841
(87) Internationale Veröffentlichungsnummer: WO 2012/113581

(56) Entgegenhaltungen:
- WO-A2-2006/123340
- US-A1- 2005 187 605
- US-A1- 2007 031 607

## Beschreibung

Die Erfindung betrifft ein Membranimplantat zur Behandlung von Gefäßfehlbildungen, das aus einem expandierbaren Stent und einer mit dem Stent verbundenen Membran besteht, wobei die Membran Maschen des Stents zumindest in einem zentralen Bereich abdeckt. Das Membranimplantat ist dazu bestimmt, endovaskulär in das zu behandelnde Gefäßsystem eines Patienten implantiert zu werden.

Zur Behandlung von vaskulären Fehlbildungen, insbesondere der Arterien, die zumeist auf ein eng begrenztes Gebiet beschränkt sind, wurde eine Vielzahl von Instrumenten entwickelt, die eine ausschließlich oder bevorzugt lokale mechanische, thermische, chemische, elektrische oder pharmakologische Wirkung zum Gegenstand haben. Diese Behandlungsmethoden beruhen zumeist auf endovaskulären Techniken, bei denen mit Hilfe eines Katheters das Behandlungsinstrument an den Einsatzort geführt und dort angewandt wird.

Eine vielfach benutzte Behandlungsform, insbesondere im Zusammenhang mit sklerotischen Verengungen von Gefäßen, aber auch zur Überbrückung von Aneurysmen oder anderen arteriovenösen Fehlbildungen ist das Setzen von Stents, mit denen die verengte Stelle aufgeweitet bzw. die Fehlbildung überbrückt wird. Zahlreiche Formen von Stents wurden entwickelt und befinden sich im Einsatz.

Dabei gibt es grundsätzlich zwei Stentformen. Zum einen sind dies die ballonaufweitbaren Stents, die zumeist aus einem medizinischen Stahl gefertigt werden und mittels eines Ballonkatheters an den Einsatzort verbracht und dort hydraulisch aufgeweitet werden. Zum anderen sind dies Stents aus selbstexpandierem Material, etwa Nitinol, die in einer kontrahierten Form durch einen Katheter an den Einsatzort vorgeschoben und dort freigesetzt werden und im freigesetzten Zustand die vorgesehene expandierte Form annehmen. Beide Stentformen können mit Medikamenten kombiniert werden, die insbesondere entzündungshemmend oder antistenotisch wirken.

Eine Stentvariante, die insbesondere zur Überbrückung von Gefäßfehlbildungen dient, ist ein mit einer Membran versehener Stent, der den Durchtritt von Blut aus dem Gefäß in die Fehlbildung erschwert oder unterbindet. Bei einem Aneurysma oder Shunt führt dies in der Regel dazu, dass sich dort ein Thrombus ausbildet, der zum Verschluss des Aneurysmas oder Shunts führt. Ein solches Membranimplantat ist beispielsweise in der WO 2010/006777 A1 beschrieben.

Ein weiteres Membranimplantat mit den Merkmalen aus dem Oberbegriff des Anspruchs 1 ist aus der US 2007/0031607 bekannt.

Stents und sonstige Implantate zur Behandlung von arteriovenösen Fehlbildungen und Stenosen werden, wie schon erwähnt, häufig mit Arzneistoffen kombiniert, um auf diese Weise über die pharmakologischen Wirkungen dieser Substanzen die rein mechanische Wirkung des Implantats zu unterstützen. So haben sich insbesondere Beschichtungen mit proliferationshemmenden Substanzen wie Paclitaxel und Rapamycin bewehrt. Zahlreiche andere Wirkstoffe sind für den Einsatz in Gefäßen im Zusammenhang mit Stents beschrieben worden, insbesondere um eine Restenose zu verhindern. Restenosen treten nach der Implantation von Stents zumeist innerhalb der ersten Wochen auf und gehen häufig auf Gefäßschädigungen zurück, die bei der Implantation des Stents entstanden sind.

Die Beschichtung von Stents und anderen Implantaten mit arzneilich wirksamen Stoffen ist außerordentlich problematisch, insbesondere was die Fixierung des Wirkstoffs auf der Stentoberfläche und die gleichmäßige Abgabe über den relevanten Zeitraum anbetrifft. Wichtig ist die Bereitstellung einer niedrigen und gleichmäßigen Wirkkonzentration über einen gewünschten Zeitraum. Da es sich bei den Wirkstoffen um höchst wirksame Mittel handelt, sind Abgabespitzen zu vermeiden und sollte die Abgabe auf den gewünschten Zeitraum beschränkt sein. Dies ist mit herkömmlichen Stentkonstruktionen und Membranen nur schwer zu verwirklichen.

Membranbeschichtete Implantate sind nicht notwendig mit Arzneimitteln beschichtet. Zur Abschottung einer Fehlbildung reicht es in der Regel aus, die jeweils zu behandelnde Fehlbildung vom Blutkreislauf abzutrennen. Hierfür wurden eine Reihe von Kunststoffmembranen entwickelt, die beispielsweise aus Polytetrafluorethylen, Polyester, Polyamid oder Polyolefinen gefertigt sind. Derartige Membranen haben aber den Nachteil, dass sie nicht ohne Weiteres mit dem sie tragenden Stent verbunden sind und quasi mechanisch über den expandierten Stent an der Gefäßwand gehalten werden. Eine Verbindung mit dem Stentgerüst selbst wäre wünschenswert. Des Weiteren wäre eine Struktur wünschenswert, die es erlaubt, die Druckwirkung des Stents bei der Expansion auf die Gefäßwand gut zu verteilen und damit das Verletzungsrisiko zu minimieren.

Aufgabe der Erfindung ist in diesem Sinne zum Einen die Bereitstellung eines Membranimplantats, das eine integrale Verbindung der Membran mit dem Stent ermöglicht, wobei die Membran geeignet ist, sowohl als "Kissen" zwischen Gefäßwand und Stent zu wirken, als auch eine Barriere zu einer Gefäßfehlbildung aufzubauen. Des Weiteren wäre es wünschenswert, eine Membran bereitzustellen, die geeignet ist, Medikamente in sich aufzunehmen und gleichmäßig über einen gewünschten Zeitraum an die benachbarte Gefäßwand abzugeben.

Hierzu weist das Membranimplantat eine Membran auf, die als Faservlies ausgebildet ist und Kunststofffibrillen enthält, wobei die Membran mit dem Stent integral verbunden und zumindest teilweise porös ausgebildet ist. Über die poröse Struktur der Membran ist diese geeignet, Arzneistoffe in sich aufzunehmen und unter physiologischen Bedingungen an die umgebende Gefäßwand abzugeben.

Die Membran des erfindungsgemäßen Membranimplantats ist ein Faservlies, das aus Kunststofffasern oder Fibrillen zusammengesetzt und direkt mit dem Stentgerüst verbunden ist. In der Regel kann das Stentgerüst selbst verwandt werden, um die Fibrillen bzw. Fasern des Faservlieses auf dem Stent abzusetzen, etwa durch Tauch- oder Sprühbeschichtung mit einer entsprechenden Lösung oder Emulsion. Die Fasern können dabei umgerichtet oder gerichtet abgesetzt werden. Die Art der Ablage wie auch die Fasergröße und die Schicktdicke haben Einfluss auf die Porosität.

Vorzugsweise wird die Membran auf dem Stent durch Elektrospinnen eines Vlieses erzeugt. Hierbei werden die Fibrillen bzw. Fasern des Faservlieses aus einer Polymerlösung mithilfe von elektrischem Strom auf einem Substrat abgeschieden. Bei der Abscheidung verkleben die Fibrillen zu einem Vlies. In der Regel haben die Fibrillen einen Durchmesser von 100 bis 1000 nm. Durch Elektrospinnen gewonnene Membranimplantate haben eine sehr gleichmäßig ausgebildete dünne Membran, die auch ohne weiteres mit dem Stentgerüst verklebt und geeignet ist, das Stentgerüst in sich einzuschließen. Die Membran ist zäh und mechanisch belastbar und kann mechanisch einfach durchstoßen werden, ohne dass die Öffnung zum Ansatzpunkt für weitergehende Risse wird. Dicke und Länge der Fibrillen wie auch der Grad der Porosität kann durch Auswahl der Verfahrensparameter gesteuert werden.

Als Material für die Membran kommt ein jedes körperverträgliches und für solche Zwecke zugelassenes Material in Frage, beispielsweise aus Polyester, Polyamid, Polyurethan, Polytetrafluorethylen oder dergleichen. Besonders bevorzugt ist Polyurethan bzw. eine Mischpolymer, das als Polycarbonaturethan bekannt ist. Im Zusammenhang mit der Schaffung der Membran und den dafür geeigneten Materialien wird insbesondere auf die WO 2008/049386, die DE 28 06 030 und die darin genannte Literatur hingewiesen.

Die erfindungsgemäß zum Einsatz kommende Membran ist zumindest teilweise porös ausgebildet. Teilweise porös bedeutet in diesem Zusammenhang, dass die der Gefäßwand zugewandte Seite (Außenseite) der Membran Poren aufweist, während die dem Gefäß zugewandte Seite (Innenseite) durch im Wesentlichen flüssigkeitsdicht ausgebildet ist.

Die Membran, wie sie erfindungsgemäß eingesetzt wird, weist in der Regel Poren in einer Größe von 1 bis 100 µm auf, insbesondere im Bereich von 10 bis 50 µm. Die Fibrillen haben einen Durchmesser von 0,5 bis 100 µm bei einer Länge von 10 µm bis 1 mm.

Eine Porengröße von etwa 20 bis 50 µm je nach Größe des Gefäßes hat sich als besonders günstig für das Einwachsen von Endothelzellen erwiesen.

Die Membran ist mehrschichtig ausgebildet, insbesondere zwei- oder dreischichtig. Die innere Schicht der Membran ist die oben genannte im Wesentlichen flüssigkeitsdichte Schicht . Dagegen ist die äußere gefäßseitige Schicht der Membran schwammförmig ausgebildet, so dass sie geeignet ist, auch einen Wirkstoff in einem geeigneten Trägerstoff aufzunehmen und durch die Poren an die Gefäßwandung abzugeben. Der Wirkstoff kann durch Tauchen oder Besprühen in die Poren eingebracht werden. Mit einer Mittelschicht aus einem dichten Faserverbund kann die Festigkeit der Membran erhöht werden.

Es ist auch eine mehrschichtige Ausführungsform möglich, bei der die einzelnen Schichten mit unterschiedlichen Verfahren hergestellt werden, beispielsweise eine innere Schicht durch Elektrospinnen, eine zweite Schicht durch Spraycoaten und eine dritte Schicht durch Elektrospinnen. Hierdurch können die Besonderheiten eines jeden Verfahrens ausgenutzt werden, insbesondere wenn es darum geht, Porosität, Festigkeit oder Dichtigkeit zu schaffen.

Der Wirkstoff kann auch in der Matrix einer Schicht eines Mehrschichtsystems eingebunden sein, d.h. auch in einer inneren Schicht. Die Freisetzung erfolgt durch Diffusion durch die Matrix hindurch oder durch Degration oder Erosion des Polymers.

In das Schichtsystem eingebunden werden können auch röntgensichtbare Substanzen, die bei der Platzierung oder Kontrolle des Implantats hilfreich sein können. Solche röntgendichten Substanzen sind beispielsweise Schwermetallsalze wie Bariumsulfat oder Jodverbindungen, wie sie üblicherweise in der Röntgentechnik als Kontrastmittel eingesetzt werden.

Ist das erfindungsgemäße Membranimplantat dazu bestimmt, die Strömung des Blutes zu beeinflussen, etwa um ein Aneurysma zu überbrücken, ist zumeist eine dünne Membran ausreichend, die auf der Innenseite im Wesentlichen flüssigkeitsdicht ausgebildet sein sollte. Auch hier ist die Außenseite porös ausgebildet, um das Einwachsen von Endothelzellen zu fördern.

Herstellungstechnisch ist es allerdings regelmäßig günstig, bei mehrschichtigen Membranen die innere wie die äußere Schicht gleich auszuführen, was Dichtigkeit, Porosität oder Festigkeit anbetrifft. Insbesondere ermöglicht dies, die Stentstruktur darin einzubetten beziehungsweise einzupacken und damit einen Verbund aus Stent und Membran zu schaffen. Die Stege des Stents sind dabei allseitig vom Membranmaterial umgeben.

Die erfindungsgemäße Membran deckt den Stent bzw. das Stentgerüst in jedem Fall in seinem zentralen Bereich ab. Zweckmäßigerweise wird es aber den gesamten Stent erfassen und abdecken, so dass der Stent über seine gesamte Länge über die Membran an die Gefäßwand zu liegen kommt.

Die Membran des Membranimplantats wird insbesondere durch Sprühbeschichtung einer entsprechenden Kunststofflösung hergestellt, wobei die Beschichtung in mehreren Arbeitsdurchgängen vorgenommen werden kann. Der Stent kann dabei als primärer Träger oder Matrix dienen, d. h. es wird direkt auf die Waben bzw. Maschen des Stents aufgesprüht, bis diese sich mit der Membran verschlossen haben. Alternativ ist es möglich, den Stent auf einen Dorn aufzuziehen und von außen zu besprühen und anschließend den Dorn wieder zu entfernen. Bei der Sprühbeschichtung kommen insbesondere für die Fasererzeugung geeignete Sprühdüsen zum Einsatz.

Eine weitere Möglichkeit besteht in der Anwendung eines sogenannten Elektrospinnverfahrens, wie es in der Fachliteratur vielfach beschrieben ist. Auch in diesem Fall werden einzelne Fasern auf der Stentoberfläche abgeschieden, die, je nach Packungsdichte, ein mehr oder weniger poröses Geflecht ergeben.

In jedem Fall ist bei der Beschichtung darauf zu achten, dass die Fasern einen hinreichend festen und dichten Verbund untereinander sowie mit dem Stentgerüst ergeben. In der Regel neigen die Fasern schon bei der Abscheidung zum Verkleben. Dies kann aber auch durch Verwendung eines Klebematerials erreicht werden, oder durch eine anschließende mechanische, thermische oder andersartige Behandlung zum Verkleben und/oder Verschweißen der einzelnen Fasern. Ultraschall kann hier zum Beispiel erfolgreich eingesetzt werden, wie auch zum "Aufbrechen" der Faserstruktur zur Erhöhung der Porigkeit. Beim Elektrospinnen können die Faserstärke, Porigkeit und das Ausmaß der Verklebung über die Geschwindigkeit gesteuert werden.

Für die Optimierung/Verbesserung der Verbundfestigkeit zwischen Faservlies und Stentgerüst kann eine haftvermittelnde Schicht zumindest außen auf das Stentgerüst aufgebracht werden. Hierbei kommen Tauch- und Sprühverfahren mit Polymeren oder Abscheidungsverfahren wie das Verfahren der Chemical Vapor Deposition (CVD) mit Parylene in Frage.

Die Membran auf dem Stentgerüst hat in der Regel eine Schichtdicke von 10 bis 400 µm, insbesondere von 10 bis 100 µm und besonders bevorzugt von 10 bis 40 µm. Die Schichtdicke ist dabei vom Einsatzzweck abhängig. Membranen, die Medikamentformulierungen in sich aufnehmen sollen, haben in der Regel eine größere Schichtdicke (und Porosität) als solche, die lediglich eine Sperrwirkung entfalten sollen.

Bei der Ausbildung der Membran kann es sinnvoll sein, mehrere Schichten mit jeweils unterschiedlich ausgerichteten Fasern vorzusehen. Hierdurch kann zum einen die Porosität beeinflusst werden, zum anderen aber auch Einfluss auf die Flexibilität und Reibungseigenschaften genommen werden. So kann z. B. durch gezielte Ausrichtung der Fasern auf der Außenseite der Membran die Gleitfähig im Katheter positiv beeinflusst werden, insbesondere durch eine parallele Ausrichtung in der Längsrichtung des Implantats. Dies erleichtert den Vorschub des Implantats im Katheter bei der Applikation.

Es ist bekannt, dass die Verwendung von Polycarbonaturethan die Membran außerordentlich körperverträglich macht. Die Porosität erlaubt zudem das Einwachsen von Endothelzellen und damit die Integration des Implantats in die Gefäßwand. Polycarbonaturethan baut sich, wie zahlreiche andere Kunststoffe, mit der Zeit im Körper ab, so dass die Membran sich nach einiger Zeit aufgelöst hat.

Der Stent des erfindungsgemäßen Membranimplantats orientiert sich hinsichtlich seines Materials und seines Designs an den an ihn gestellten Aufgaben. Wie auch die Membran kann der Stent selbstauflösend gestaltet sein, beispielsweise durch Verwendung von Reineisen, Magnesium, Magnesiumlegierungen oder Kobalt-Chrom-Legierungen.

Als Stentgerüst kann ein jeder herkömmliche zur Implantation in ein Gefäß geeignete Stent verwandt werden. Bevorzugt sind Stents, die eine relativ geringe Gerüstdichte haben, also im Wesentlichen offen sind, da der Stent in diesem Fall lediglich der Fixierung der Membran an der Gefäßoberfläche dient. Eine Stützwirkung wird nicht immer benötigt. Ist eine Stützwirkung verlangt, muss der Stent entsprechend ausgestaltet sein. Insbesondere für eine Stabilisierung der Membran ist eine erhöhte Gerüstdichte (kleine Maschen) sinnvoll.

Erfindungsgemäß können auch selbstexpandierende Stents zum Einsatz kommen, wie sie aus Formgedächtnismaterialien gefertigt werden. Bekannt sind Stents aus Nitinol oder einer ternären Nickel-Titan-Legierung, die unter dem äußeren Zwang eines Katheters in kontrahierter Form transportiert werden können, nach Verlassen des Katheters die ihnen aufgeprägte expandierte Form einnehmen und sich mit der Membran an die Gefäßwand anlegen. Solche Stents sind insbesondere für Membranimplantate geeignet, die der Strömungsbeeinflussung dienen, etwa zur Abschottung von Gefäßfehlbildungen wie Aneurysmen.

Bei den Stents kann es sich um auf übliche Weise aus einem rohrförmigen Körper geschnittene Stents handeln, aber auch um sogenannte geflochtene Stents aus entsprechendem Drahtmaterial. Insbesondere letztere können als "Neurostents" zur Überbrückung von Aneurysmen oder anderen Gefäßfehlbildungen im Gehirn eingesetzt werden.

Die Verwendung von aus Draht gefertigten Stents oder Stents mit geringer Gerüstdichte in Verbindung mit den flexiblen Membranen aus Faservlies erlaubt die Fertigung von außerordentlich flexiblen und dünnen Membranimplantaten, die auch in kleinlumige Gefäße problemlos eingebracht werden können. Solche flexiblen Stents für kleinlumige Gefäße kommen insbesondere in der Neuroradiologie zum Einsatz.

Erfindungsgemäß kann das Membranimplantat auch aus einer Kombination von zwei außen liegenden Stents mit einer dazwischenliegenden Membran aus Faservlies bestehen. Gleichermaßen ist es möglich, dass Stentgerüst nur auf der Außenseite oder nur auf der Innenseite mit der Membran aus Faservlies zu versehen. Schließlich ist es auch möglich, das erfindungsgemäße Membranimplantat so herzustellen, dass ein innen liegender Stent allseitig von der Membran umgeben ist, so dass ein Kontakt sowohl der Gefäßwand als auch des Blutes mit dem Stentgerüst unterbunden wird. Erfindungsgemäß ist ein solcher beidseitig von Faservlies bedeckter Stent bevorzugt. Dies bedeutet, dass die Stege des Stents in das Faservlies eingebettet und von dem Vlies umgeben sind.

Für den Fall, dass das erfindungsgemäße Membranimplantat mit einem Medikament beladen werden soll, wird dieses vorzugsweise in die äußere gefäßseitige Schicht der Membran eingebracht. Diese ist hierzu schwammförmig ausgebildet. Das Medikament wird dann zunächst in größeren Mengen unter dem Expansionsdruck "ausgepresst", danach aber über einen gewissen Zeitraum gleichmäßig an die Gefäßwandung abgegeben. Für die lokale Anwendung sind je nach Art der Erkrankung sehr unterschiedliche Arzneistoffe geeignet. Zur Prophylaxe und Therapie von Gefäßverengungen sowie andere überwiegend raumfordernder Prozesse werden zytostatisch wirksame Substanzen eingesetzt, wie sie beispielsweise in der Tumortherapie oder zur Restenoseprophylaxe auf Stents oder Ballonkathetern verwendet werden.

Zur Behandlung von wenig oder nicht-stenosierenden, beispielsweise entzündlichen, lipidreichen oder infektiösen Gefäßwandveränderungen können Zytostatika wegen ihrer entzündungshemmenden Eigenschaften, Immunsuppressiva, steroidale und nicht-steroidale Entzündungshemmer, Statine, Antioxidianzien, Gerinnungshemmer oder Gemische einzelner Wirkstoffe eingesetzt werden. Eine Vielzahl derartiger Substanzen sind in Lehrbüchern der Pharmakologie und zahlreichen Patentschriften beschrieben, z. B. in der US 2008/0118544 oder DE 10 2007 036 685. Bevorzugte Wirkstoffe sind Paclitaxel, Docitaxel, Protaxel und andere Taxane, Methotrexat, 2-Desoxyglukose, Thalidomid, Triamcinolon, Betamethason, Dexamethason und deren Derivate, Genistein, Sirolimus, Everolimus und andere mTOR-Inhibitoren, Artorvastatin, Cerivastatin, Fluvastatin, Lovastatin, Pitavastatin, Pravastatin, Rosuvastatin, Simvastatin, Doxycyclin, Minucyclin, Probucol, Tocopherol, Ascorbinsäure, Arsentrioxid und andere Arsensalze und -verbindungen, Wismutsalze und -verbindungen. Soweit anwendbar sind die betreffenden Wirkstoffe auch als Salze oder Komplexverbindungen oder kovalent gebunden, z. B. in Form von Prodrugs oder ihrer ihrerseits wirksamen Derivate einsetzbar. Soweit erforderlich, können Mischungen bestimmter Wirkstoffe eingesetzt werden, wobei jeder Wirkstoff entsprechend seiner Wirkstärke dosiert wird.

Das erfindungsgemäße Membranimplantat wird über einen üblichen Katheter an seinen Einsatzort transportiert. Soweit es ein ballonexpandierender Stent ist, ist dieser in üblicher Weise mit der Membran auf einen Ballon aufgekrimpt. Im Falle eines selbstexpandierenden Stents ist der membranbehaftete Stent über einen Führungsdraht durch einen Katheter platzierbar. Die hier zum Einsatz kommenden Techniken sind dem Fachmann allgemein bekannt.

Die erfindungsgemäßen Membranimplantate können durch übliche Tauch- und Sprühverfahren hergestellt werden, bei denen das Stentgerüst in einer Reihe von Schritten mit der Membran versehen werden. Bevorzugt ist aber ein Elektrospinnverfahren, bei dem eine Lösung des für die Membran verwandten Kunststoffes in einem organischen Lösungsmittel, etwa Polycarbonaturethan in Chloroform, zum Einsatz kommt. Bei diesem Verfahren wird zunächst das später innen liegende Vlies auf einem Kern abgeschieden und anschließend der Stent auf den Kern geschoben. Danach wird der Kern erneut eingespannt und noch einmal besponnen. Die Parameter der beiden Elektrospinnschritte können gleich sein, aber auch variieren, beispielsweise um die Außenschicht dicker und stärker porös auszugestalten. Die Außenschicht ist die Schicht, die auf der Gefäßwand zu liegen kommt und gegebenenfalls mit Arznei beladen wird.

Als Dorn wird in der Regel ein Edelstahlkern verwandt, der später aus dem Konstrukt herausgezogen wird. Je nach Spinngeschwindigkeit kommt es zu einem mehr oder weniger starken Verbund der Innen- mit der Außenschicht durch die Maschen des Stents hindurch. Eine hohe Spinngeschwindigkeit ist für diesen Verbund förderlich: Der dann höhere Lösungsmittelgehalt der abgeschiedenen Fibrillen führt zu einer stärkeren Verklebung mit bereits versponnenem Material.

Die Erfindung wird durch die beiliegenden Abbildungen näher erläutert. Es zeigen:
- Figur 1: eine Schnittdarstellung eines erfindungsgemäßen Membranimplantats mit zweischichtigem Aufbau der Membran;
- Figur 2: im Detail ein erfindungsgemäßes Membranimplantat mit einem dreischichtigen Aufbau der Membran;
- Figur 3: im Detail ein erfindungsgemäßes Membranimplantat mit einem zweischichtigen Aufbau der Membran, die auf die Innenseite und auf die Außenseite des Stents aufgebracht ist;
- Figur 4: eine zeichnerische Darstellung eines erfindungsgemäßen Membran-implantats ;
- Figur 5: eine fotografische Darstellung eines erfindungsgemäßen Membran-implantats; und
- Figur 6: ein vergrößerter Auschnitt eines Membranimplantats gemäß Figur 5.

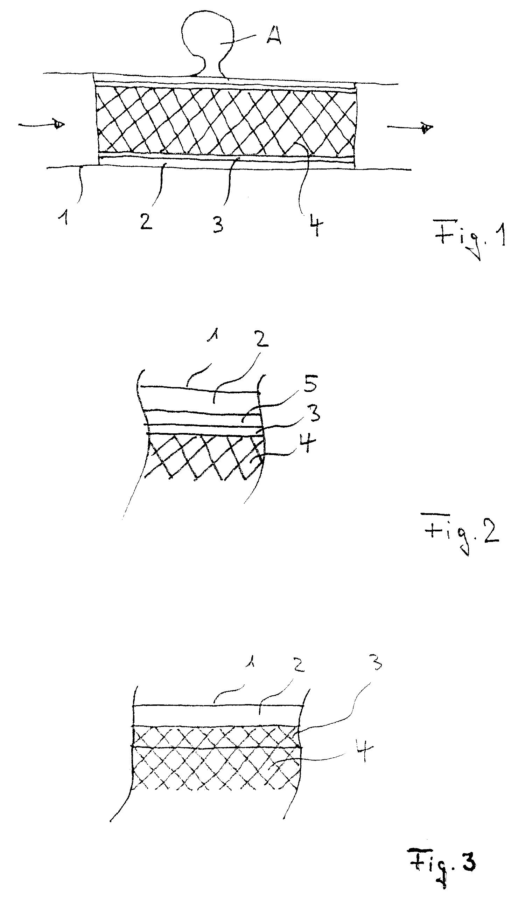

Abbildung 1 zeigt ein in einem Blutgefäß 1 angeordnetes erfindungsgemäßes Membranimplantat, dessen Membran eine äußere schwammförmige Schicht 2 aufweist, die sich an die Gefäßwand anlegt, eine innere, mehr oder weniger flüssigkeitsdichte Schicht 3, die verhindern soll, dass das durch das Gefäß strömende Blut (Pfeilrichtung) durch die Membran hindurchtritt sowie die Stütztstruktur des Stents 4, die dazu dient, die Membran an die Gefäßwand 1 zu pressen. Die Membran dient beispielsweise dazu, ein Aneurysma A mit beerenförmiger Struktur vom Blutkreislauf abzuschotten und damit dessen Okklusion herbeizuführen. Der Stent kann aber mit der schwammförmigen Außenschicht der Membran 2 auch Medikamente aufnehmen und damit beispielsweise eine antistenotische Wirkung auf die Wand von dilatierten Gefäßen ausüben.

Figur 2 zeigt einen Ausschnitt aus der Wandstruktur eines erfindungsgemäßen Membranimplantats mit dem Stent 4 auf der Innenseite, eine daran anschließende Schicht 3, die im Wesentlichen flüssigkeitsdicht ist, einer festen Zwischenschicht 5 und einer schwammförmigen Außenschicht 2, die an die Gefäßwand 1 zum Liegen kommt.

Es versteht sich, dass der Stent mit seiner Gerüststruktur 4 in die Membran integriert sein kann, so dass die innere glatte Schicht der Membran auf der Innenseite des Stents angeordnet ist und dort eine vergleichsweise glatte Wandung des Implantats erzeugt, die der Blutströmung wenig Widerstand entgegensetzt.

Figur 3 zeigt einen Ausschnitt aus der Wandstruktur eines erfindungsgemäßen Membranimplantats mit dem Stentgerüst 4, einer Membranschicht auf der Innenseite des Stentgerüsts 4, einer zweiten Membranschicht auf der Außenseite, wobei die Schichten 2 und 3 durch die Maschen hindurch miteinander verbunden sind sowie die Gefäßwand 1.

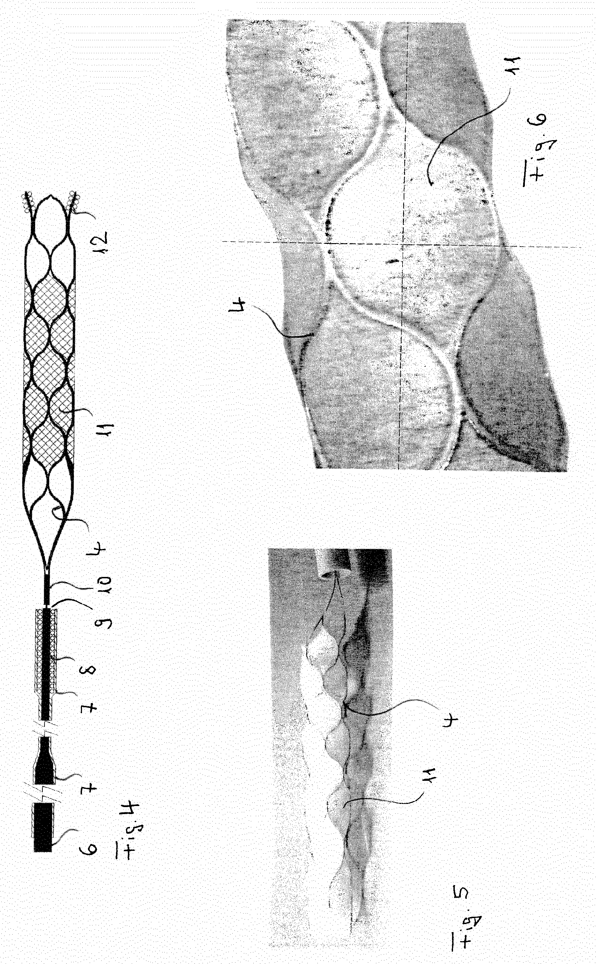

Figur 4 zeigt ein erfindungsgemäßes Membranimplantat mit dem Stentgerüst 4, dass über ein Kupplungselement 10 mit einem Führungsdraht 6 verbunden ist. Der Führungsdraht 6 ist in seinem distalen Bereich mit einem Kunststoffschlauch 7 versehen, der eine Markerspirale 8 abdeckt. Eine Ablösestelle 9 dient der Trennung des Implantats vom Führungsdraht, im dargestellten Fall durch einen an und für sich bekannten elektrolytischen Prozess. Alternativ können mechanische Ablösungssysteme vorgesehen sein.

Das Stentgerüst 4 des Membranimplantats ist im zentralen Bereich mit einer Membran 11 versehen, die die Maschenstruktur abdeckt. Im distalen Bereich des Implantats finden sich Markerspiralen 12, die die exakte Platzierung des Implantats ermöglichen.

Im dargestellten Fall handelt es sich bei dem Stent 4 um einen aus einem Metallrohr geschnittenen Stent, der in komprimierter Form in einem Katheter geführt werden kann und sich nach Herausschieben aus dem Katheter entfaltet. Es kommen insbesondere Formgedächtnismaterialen in Frage, etwa Nitinol. Alternativ können aber auch geflochtene Stentstrukturen verwandt werden, insbesondere solche aus Nitinolfilamenten.

Figur 5 ist die fotografische Darstellung eines erfindungsgemäßen Membranimplantats nach dem Austritt aus einem Katheterrohr. Deutlich zu erkennen sind die Drahtmaschen 4 mit der Membranbespannung 11.

Figur 6 zeigt in fotografischer Vergrößerung ein erfindungsgemäßes Membranimplantat, wie schon in Figur 4 und 5 gezeigt, mit den Stegen des Gerüsts 4 und der Membranbespannung, die in diesem Fall die Stege einschließt.

## Patentansprüche

1. Membranimplantat zur Behandlung von Gefäßfehlbildungen, das endovaskulär in das zu behandelnde Gefäß implantierbar ist, wobei das Membranimplantat aus einem expandierbaren Stent (4) und einer mit dem Stent (4) verbundenen Membran (2, 3, 5, 11) besteht, die Membran (2, 3, 5, 11) Maschen des Stents zumindest in einem zentralen Bereich abdeckt, die Membran (2, 3, 5, 11) als Faservlies ausgebildet ist, das Kunststofffibrillen enthält und mit dem Stent (4) einen Verbund bildet und zumindest teilweise porös ausgebildet ist,
**dadurch gekennzeichnet, dass** die Membran (2, 3, 5, 11) eine äußere gefäßseitige Schicht aufweist, die schwammförmig ausgebildet ist und die innere Schicht (3) im wesentlichen flüssigkeitsdicht und glatt ist.

2. Membranimplantat nach Anspruch 1, **dadurch gekennzeichnet, dass** die Membran (2, 3, 5, 11) mehrschichtig, insbesondere zwei- oder dreischichtig, ausgebildet ist.

3. Membranimplantat nach Anspruch 1 oder 2 mit einer Membran (2, 3, 5, 11) aus Polycarbonaturethan.

4. Membranimplantat nach einem der Ansprüche 1 bis 3, erhältlich durch Elektrospinnen der Membran (2, 3, 5, 11).

5. Membranimplantat nach Anspruch 4, erhältlich durch ein mehrstufiges Elektrospinnverfahren, bei dem in einem ersten Schritt auf einem Kern eine Innenmembran (3) erzeugt wird, darauf der Stent (4) platziert wird und anschließend die Außenmembran (2) unter Anbindung an die Innenmembran (3) aufgebracht wird, wonach das Implantat vom Kern getrennt wird.

6. Membranimplantat nach einem der vorstehenden Ansprüche, **dadurch gekennzeichnet, dass** die Membran (2, 3, 5, 11) die Stege des Stents (4) allseitig einschließt.

7. Membranimplantat nach einem der vorstehenden Ansprüche, **dadurch gekennzeichnet, dass** die Membran (2, 3, 5) eine Schichtdicke von 10 bis 100 µm, insbesondere 10 bis 400 µm aufweist.

8. Membranimplantat nach einem der vorstehenden Ansprüche, **gekennzeichnet durch** Poren einer Größe von 1 bis 100 µm, insbesondere 10 bis 50 µm, in der Membran.

9. Membranimplantat nach einem der Ansprüche 1 bis 7, **dadurch gekennzeichnet, dass** der Stent (4) ein selbstexpandierender Stent aus einem Formgedächtnismaterial ist.

10. Membranimplantat nach Anspruch 9, **dadurch gekennzeichnet, dass** der Stent aus Nitinol oder einer ternären Nickeltitan-Legierung besteht.

11. Membranimplantat nach einem der vorstehenden Ansprüche, **dadurch gekennzeichnet, dass** die Membran (2, 3, 5, 11) zumindest in ihrer äußeren Schicht (2) mit einem Medikament beladen ist.

12. Membranimplantat nach Anspruch 13, **dadurch gekennzeichnet, dass** das Medikament entzündungshemmend oder antistenotisch wirkt.

13. Membranimplantat nach einem der vorstehenden Ansprüche, **dadurch gekennzeichnet, dass** die Membran (2, 3, 5, 11) mit einem Röntgenkontrastmittel beladen ist.

14. Membranimplantat nach einem der vorstehenden Ansprüche, **dadurch gekennzeichnet, dass** der Stent (4) eine haftvermittelnde Schicht zumindest auf der Außenseite aufweist.

## Claims

1. Membrane implant for the treatment of vascular malformations, said implant being implantable by endovascular methods into the vessel to be treated, wherein the membrane implant consisting of an expandable stent (4) and a membrane (2, 3, 5, 11) connected with the stent (4), the membrane implant covering said membrane (2, 3, 5, 11) meshes of the stent at least in a central region, the membrane (2, 3, 5, 11) is provided in the form of a non-woven fabric comprising plastic fibrils, the membrane (2, 3, 5, 11) forming a bond with the stent (4) and, at least partially, being of porous design,
**characterized that** the membrane (2, 3, 5, 11) has an outer layer facing the wall of the vessel and said layer having a sponge-like structure and the inner layer (3) is substantially of liquid-tight and smooth design.

2. Membrane implant according to claim 1, **characterized in that** the membrane (2, 3, 5, 11) is multi-layered, in particular of two- or three-layer design.

3. Membrane implant according to claims 1 or 2 with a membrane (2, 3, 5, 11) of polycarbonate urethane.

4. Membrane implant according to any one of claims 1 to 3 obtained by means of electrospinning of the membrane (2, 3, 5, 11).

5. Membrane implant according to claim 4 obtained by a multi-stage electrospinning process, wherein in a first step an inner membrane (3) is produced on a core, with the stent (4) mounted on the membrane and, subsequently, the outer membrane (2) being arranged and bonded to the inner membrane (3), after which the implant is separated from the core.

6. Membrane implant according to any one of the preceding claims, **characterized in that** the membrane (2, 3, 5, 11) embraces the webs of the stent (4) on all sides.

7. Membrane implant according to any one of the preceding claims, **characterized in that** the membrane (2, 3, 5) has a layer thickness of between 10 and 100 µm, in particular between 10 and 400 µm.

8. Membrane implant according to any one of the preceding claims, **characterized by** pores of a size ranging between 1 and 100 µm, in particular between 10 and 50 µm, in the membrane.

9. Membrane implant according to any one of the claims 1 to 7, **characterized in that** the stent (4) is a self-expanding stent made of a shape-memory material.

10. Membrane implant according to claim 9, **characterized in that** the stent consists of nitinol or a ternary nickel-titanium alloy.

11. Membrane implant according to any one of the preceding claims, **characterized in that** the membrane (2, 3, 5, 11) accommodates a medical substance at least in its outer layer (2).

12. Membrane implant according to claim 11, **characterized in that** the medical substance has an anti-inflammatory or anti-stenotic effect.

13. Membrane implant according to any one of the preceding claims, **characterized in that** the membrane (2, 3, 5, 11) accommodates a radiopaque contrast medium.

14. Membrane implant according to any one of the preceding claims, **characterized in that** the stent (4) is provided at least on the outside with an adhesion-promoting layer.

## Revendications

1. Implant membranaire pour le traitement de malformations vasculaires, qui peut être implanté par voie endovasculaire dans le vaisseau à traiter, l'implant membranaire étant constitué d'un stent expansible (4) et d'une membrane (2, 3, 5, 11) liée au stent (4), la membrane (2, 3, 5, 11) recouvrant les mailles du stent au moins dans une zone centrale, la membrane (2, 3, 5, 11) étant configurée comme un non-tissé contenant des fibrilles d'un matériau plastique et formant un composite avec le stent (4) et ayant au moins partiellement une structure poreuse, **caractérisé en ce que** la membrane (2, 3, 5, 11) comporte une couche extérieure côté vaisseau, qui a une structure spongieuse, et que la couche intérieure (3) est pour l'essentiel étanche aux liquides et lisse.

2. Implant membranaire selon la revendication 1, **caractérisé en ce que** la membrane (2, 3, 5, 11) a une structure multicouche, en particulier bi- ou tricouche.

3. Implant membranaire selon la revendication 1 ou 2, comportant une membrane (2, 3, 5, 11) en polycarbonate-uréthanne.

4. Implant membranaire selon l'une des revendications à 3, pouvant être obtenue par électrofilage de la membrane (2, 3, 5, 11).

5. Implant membranaire selon la revendication 4, pouvant être obtenu par un procédé d'électrofilage en plusieurs étapes, dans lequel, dans une première étape, une membrane intérieure (3) est produite sur un noyau, le stent (4) est placé par-dessus, puis la membrane extérieure (2) est appliquée par liaison à la membrane intérieure (3), ce après quoi l'implant est séparé du noyau.

6. Implant membranaire selon l'une des revendications précédentes, **caractérisé en ce que** la membrane (2, 3, 5, 11) entoure sur tous les côtés les traverses du stent (4).

7. Implant membranaire selon l'une des revendications précédentes, **caractérisé en ce que** la membrane (2, 3, 5) présente une épaisseur de couche de 10 à 100 µm, en particulier de 10 à 400 µm.

8. Implant membranaire selon l'une des revendications précédentes, **caractérisé par** des pores ayant une grosseur de 1 à 100 µm, en particulier de 10 à 50 µm, dans la membrane.

9. Implant membranaire selon l'une des revendications 1 à 7, **caractérisé en ce que** le stent (4) est un stent auto-expansible en un matériau à mémoire de forme.

10. Implant membranaire selon la revendication 9, **caractérisé en ce que** le stent est constitué de Nitinol ou d'un alliage ternaire de nickel-titane.

11. Implant membranaire selon l'une des revendications précédentes, **caractérisé en ce que** la membrane (2, 3, 5, 11) est, au moins dans sa couche extérieure (2), chargée d'un médicament.

12. Implant membranaire selon la revendication 13, **caractérisé en ce que** le médicament a un effet anti-inflammatoire ou antisténotique.

13. Implant membranaire selon l'une des revendications précédentes, **caractérisé en ce que** la membrane (2, 3, 5, 11) est chargée d'un produit de contraste radiographique.

14. Implant membranaire selon l'une des revendications précédentes, **caractérisé en ce que** le stent (4) comprend une couche promotrice d'adhérence au moins sur le côté extérieur.
